# EUROPEAN PATENT APPLICATION

(11) **EP 1 222 928 A2**
(43) Date of publication of application: **17.07.2002**
(21) Application number: 02000185.5
(22) Date of filing: 09.01.2002
(51) Int. Cl.: A61K 39/00, A61P 35/00

(54) **Pharmaceutical compositions for treating or preventing cancer, especially melanoma**

(30) Priority: 16.01.2001 EP 01100914
(71) Applicant: Universität Zürich Institut für Medizinische Virologie, 8028 Zürich (CH)
(72) Inventor: Mölling, Karin, 8044 Zürich (CH); Nawrath, Michael, 8032 Zürich (CH); Pavlovic, Jovan, 8044 Zürich (CH)
(74) Representative: VOSSIUS & PARTNER

(57) **Abstract**

The present invention relates to a pharmaceutical or vaccine composition comprising a nucleic acid molecule encoding a tumor-associated antigen and at least. one peptide comprising a region corresponding to a putative cytotoxic T cell, helper T cell or B cell epitope of a tumor-associated antigen and/or cells pulsed with such peptides, optionally in combination with a pharmaceutically acceptable carrier. Such pharmaceutical compositions can be used for the treatment of cancer or for the vaccination against cancer.

SEQUENCE LISTING

<110> Unitectra Inc.

<120> Pharmaceutical Compositions for Treating or Preventing
      Cancer

<130> E 2862 EP

<140>
<141>

<160> 13

<170> PatentIn Ver. 2.1

<210> 1
<211> 1881
<212> DNA
<213> Mus musculus

<220>
<221> CDS
<222> (1)..(1881)

<400> 1
atg gtg ggt gtc cag aga agg agc ttc ctt ccc gtg ctt gtg ctg agt    48
Met Val Gly Val Gln Arg Arg Ser Phe Leu Pro Val Leu Val Leu Ser
  1               5                  10                  15

gct ctg ctg gct gtg ggg gcc cta gaa gga tcc agg aat cag gac tgg    96
Ala Leu Leu Ala Val Gly Ala Leu Glu Gly Ser Arg Asn Gln Asp Trp
             20                  25                  30

ctt ggt gtc cca aga caa ctt gta act aaa acc tgg aac agg cag ctg    144
Leu Gly Val Pro Arg Gln Leu Val Thr Lys Thr Trp Asn Arg Gln Leu
         35                  40                  45

tac ccc gag tgg aca gag gtg cag ggg tct aac tgc tgg aga ggt ggc    192
Tyr Pro Glu Trp Thr Glu Val Gln Gly Ser Asn Cys Trp Arg Gly Gly
     50                  55                  60

cag gta tct ctg agg gtc att aat gat ggg cct aca ctg gtt ggt gca    240
Gln Val Ser Leu Arg Val Ile Asn Asp Gly Pro Thr Leu Val Gly Ala
 65                  70                  75                  80

aat gcc tcc ttt tcc att gcc ctg cac ttc cct gga agt caa aag gta    288
Asn Ala Ser Phe Ser Ile Ala Leu His Phe Pro Gly Ser Gln Lys Val
                 85                  90                  95

cta ccg gat ggt cag gtt atc tgg gcc aac aac acc atc atc aat ggg    336
Leu Pro Asp Gly Gln Val Ile Trp Ala Asn Asn Thr Ile Ile Asn Gly
            100                 105                 110

agc cag gtg tgg gga gga cag cca gtg tat cca cag gag cct gat gat    384
Ser Gln Val Trp Gly Gly Gln Pro Val Tyr Pro Gln Glu Pro Asp Asp
        115                 120                 125

gcc tgt gtc ttc cct gac ggt gga ccc tgc cca tct ggt cct aaa cct    432

Ala Cys Val Phe Pro Asp Gly Gly Pro Cys Pro Ser Gly Pro Lys Pro
    130                 135                 140

ccg aag aga agc ttt gtt tat gtt tgg aag acc tgg gga aaa tac tgg 480
Pro Lys Arg Ser Phe Val Tyr Val Trp Lys Thr Trp Gly Lys Tyr Trp
145                 150                 155                 160

caa gtt ctg ggg ggt cca gtg tcc agg tcg agc att gct acg cgc cac 528
Gln Val Leu Gly Gly Pro Val Ser Arg Ser Ser Ile Ala Thr Arg His
                165                 170                 175

gca aag ctg ggc aca cac aca atg gaa gtg act gtc tac cac cga cgg 576
Ala Lys Leu Gly Thr His Thr Met Glu Val Thr Val Tyr His Arg Arg
            180                 185                 190

ggt tcc cag agc tac gtg ccc ctt gct cac gcc agt tca acc ttc acc 624
Gly Ser Gln Ser Tyr Val Pro Leu Ala His Ala Ser Ser Thr Phe Thr
        195                 200                 205

att act gac cag gta cct ttc tcc gtg agt gtg tcc cag cta cag gcc 672
Ile Thr Asp Gln Val Pro Phe Ser Val Ser Val Ser Gln Leu Gln Ala
    210                 215                 220

ttg gac gga gag acc aag cac ttc ctg aga aat cat cct ctc atc ttt 720
Leu Asp Gly Glu Thr Lys His Phe Leu Arg Asn His Pro Leu Ile Phe
225                 230                 235                 240

gcc ctt cag ctc cac gac ccc agt ggt tat ttg gcc gag gcc gac ctc 768
Ala Leu Gln Leu His Asp Pro Ser Gly Tyr Leu Ala Glu Ala Asp Leu
                245                 250                 255

tcc tac aca tgg gac ttt gga gat ggt act ggg acc ctg atc tct cgg 816
Ser Tyr Thr Trp Asp Phe Gly Asp Gly Thr Gly Thr Leu Ile Ser Arg
            260                 265                 270

gca ctt gat gtc act cac act tac ctg gag tcg ggc tca gtc act gcc 864
Ala Leu Asp Val Thr His Thr Tyr Leu Glu Ser Gly Ser Val Thr Ala
        275                 280                 285

cag gtg gta ctg cag gct gcc att cct ctt gtt tcc tgt ggt tcc tcc 912
Gln Val Val Leu Gln Ala Ala Ile Pro Leu Val Ser Cys Gly Ser Ser
    290                 295                 300

cca gtc ccg ggt acc aca gat ggc tac atg cca act gca gaa gca cct 960
Pro Val Pro Gly Thr Thr Asp Gly Tyr Met Pro Thr Ala Glu Ala Pro
305                 310                 315                 320

gga acc aca tct agg caa gga acc act aca aaa gtt gtg ggt act aca 1008
Gly Thr Thr Ser Arg Gln Gly Thr Thr Thr Lys Val Val Gly Thr Thr
                325                 330                 335

cct ggc cag atg cca act aca cag ccc tct gga acc aca gtt gta caa 1056
Pro Gly Gln Met Pro Thr Thr Gln Pro Ser Gly Thr Thr Val Val Gln
            340                 345                 350

atg cca acc aca gag gtc aca gct act aca tct gag cag atg ctg acc 1104
Met Pro Thr Thr Glu Val Thr Ala Thr Thr Ser Glu Gln Met Leu Thr
        355                 360                 365

tca gcg gtc ata gat acc aca ctg gca gag gtg tca act aca gag ggt 1152
Ser Ala Val Ile Asp Thr Thr Leu Ala Glu Val Ser Thr Thr Glu Gly
    370                 375                 380

aca ggt acc aca ccc aca agg cct tct gga acc acc gtt gca caa gca 1200
Thr Gly Thr Thr Pro Thr Arg Pro Ser Gly Thr Thr Val Ala Gln Ala
385                 390                 395                 400

aca acc aca gag ggt cca gat gcc agc cca ttg ctg ccc aca caa agt 1248
Thr Thr Thr Glu Gly Pro Asp Ala Ser Pro Leu Leu Pro Thr Gln Ser
                405                 410                 415

tct aca ggg tcc att agc cct cta ctg gat gac acc gac acc ata atg 1296
Ser Thr Gly Ser Ile Ser Pro Leu Leu Asp Asp Thr Asp Thr Ile Met
            420                 425                 430

ctt gtg aag aga caa gtt ccc ctg gac tgt gtt cta tat cga tat ggt 1344
Leu Val Lys Arg Gln Val Pro Leu Asp Cys Val Leu Tyr Arg Tyr Gly
        435                 440                 445

tct ttc tcc ctc gcc ctg gac att gtc cag ggt att gaa agt gct gag 1392
Ser Phe Ser Leu Ala Leu Asp Ile Val Gln Gly Ile Glu Ser Ala Glu
    450                 455                 460

atc ctg cag gct gtg cca ttc agt gaa ggg gat gca ttt gag ctg act 1440
Ile Leu Gln Ala Val Pro Phe Ser Glu Gly Asp Ala Phe Glu Leu Thr
465                 470                 475                 480

gtg tcc tgc caa ggc ggg cta ccc aag gaa gcc tgt atg gac att tca 1488
Val Ser Cys Gln Gly Gly Leu Pro Lys Glu Ala Cys Met Asp Ile Ser
                485                 490                 495

tca cca ggg tgc cag ccc cct gcc cag agg ctg tgc cag tct gtt cca 1536
Ser Pro Gly Cys Gln Pro Pro Ala Gln Arg Leu Cys Gln Ser Val Pro
            500                 505                 510

ccg agc cca gac tgc cag ctg gtt cta cac caa gtg ctg aaa ggt ggc 1584
Pro Ser Pro Asp Cys Gln Leu Val Leu His Gln Val Leu Lys Gly Gly
        515                 520                 525

tca ggg aca tat tgc ctc aat gtg tct ttg gct gac gcc aac agc ctg 1632
Ser Gly Thr Tyr Cys Leu Asn Val Ser Leu Ala Asp Ala Asn Ser Leu
    530                 535                 540

gca gtg gcc agc acc caa ctt gtt gtt cct ggt caa gac ggt ggc ctt 1680
Ala Val Ala Ser Thr Gln Leu Val Val Pro Gly Gln Asp Gly Gly Leu
545                 550                 555                 560

ggg cag gct ccc ttg ctt gta ggt atc ttg ctg gtg ttg gtg gct gtg 1728
Gly Gln Ala Pro Leu Leu Val Gly Ile Leu Leu Val Leu Val Ala Val
                565                 570                 575

gtc ctt gca tct ctg ata cta ggc ata gac tta aga agc agg gct cag 1776
Val Leu Ala Ser Leu Ile Leu Gly Ile Asp Leu Arg Ser Arg Ala Gln
            580                 585                 590

ttt ccc aaa tgc cac atg gta gca ctc act gct gcg cct gcc tcc ggt 1824
Phe Pro Lys Cys His Met Val Ala Leu Thr Ala Ala Pro Ala Ser Gly
        595                 600                 605

ctt cgc gcc cgc ggc ctt gga gaa aac agc ccg ctc ctc agt gga cag    1872
Leu Arg Ala Arg Gly Leu Gly Glu Asn Ser Pro Leu Leu Ser Gly Gln
    610                 615                 620

cag gtc tga                                                        1881
Gln Val
625

<210> 2
<211> 626
<212> PRT
<213> Mus musculus

<400> 2
Met Val Gly Val Gln Arg Arg Ser Phe Leu Pro Val Leu Val Leu Ser
  1               5                  10                  15

Ala Leu Leu Ala Val Gly Ala Leu Glu Gly Ser Arg Asn Gln Asp Trp
             20                  25                  30

Leu Gly Val Pro Arg Gln Leu Val Thr Lys Thr Trp Asn Arg Gln Leu
         35                  40                  45

Tyr Pro Glu Trp Thr Glu Val Gln Gly Ser Asn Cys Trp Arg Gly Gly
     50                  55                  60

Gln Val Ser Leu Arg Val Ile Asn Asp Gly Pro Thr Leu Val Gly Ala
 65                  70                  75                  80

Asn Ala Ser Phe Ser Ile Ala Leu His Phe Pro Gly Ser Gln Lys Val
                 85                  90                  95

Leu Pro Asp Gly Gln Val Ile Trp Ala Asn Asn Thr Ile Ile Asn Gly
            100                 105                 110

Ser Gln Val Trp Gly Gly Gln Pro Val Tyr Pro Gln Glu Pro Asp Asp
        115                 120                 125

Ala Cys Val Phe Pro Asp Gly Gly Pro Cys Pro Ser Gly Pro Lys Pro
    130                 135                 140

Pro Lys Arg Ser Phe Val Tyr Val Trp Lys Thr Trp Gly Lys Tyr Trp
145                 150                 155                 160

Gln Val Leu Gly Gly Pro Val Ser Arg Ser Ser Ile Ala Thr Arg His
                165                 170                 175

Ala Lys Leu Gly Thr His Thr Met Glu Val Thr Val Tyr His Arg Arg
            180                 185                 190

Gly Ser Gln Ser Tyr Val Pro Leu Ala His Ala Ser Ser Thr Phe Thr
        195                 200                 205

Ile Thr Asp Gln Val Pro Phe Ser Val Ser Val Ser Gln Leu Gln Ala
    210                 215                 220

Leu Asp Gly Glu Thr Lys His Phe Leu Arg Asn His Pro Leu Ile Phe

225                 230                 235                 240

Ala Leu Gln Leu His Asp Pro Ser Gly Tyr Leu Ala Glu Ala Asp Leu
                245                 250                 255

Ser Tyr Thr Trp Asp Phe Gly Asp Gly Thr Gly Thr Leu Ile Ser Arg
            260                 265                 270

Ala Leu Asp Val Thr His Thr Tyr Leu Glu Ser Gly Ser Val Thr Ala
        275                 280                 285

Gln Val Val Leu Gln Ala Ala Ile Pro Leu Val Ser Cys Gly Ser Ser
    290                 295                 300

Pro Val Pro Gly Thr Thr Asp Gly Tyr Met Pro Thr Ala Glu Ala Pro
305                 310                 315                 320

Gly Thr Thr Ser Arg Gln Gly Thr Thr Thr Lys Val Val Gly Thr Thr
                325                 330                 335

Pro Gly Gln Met Pro Thr Thr Gln Pro Ser Gly Thr Thr Val Val Gln
            340                 345                 350

Met Pro Thr Thr Glu Val Thr Ala Thr Thr Ser Glu Gln Met Leu Thr
        355                 360                 365

Ser Ala Val Ile Asp Thr Thr Leu Ala Glu Val Ser Thr Thr Glu Gly
    370                 375                 380

Thr Gly Thr Thr Pro Thr Arg Pro Ser Gly Thr Thr Val Ala Gln Ala
385                 390                 395                 400

Thr Thr Thr Glu Gly Pro Asp Ala Ser Pro Leu Leu Pro Thr Gln Ser
                405                 410                 415

Ser Thr Gly Ser Ile Ser Pro Leu Leu Asp Asp Thr Asp Thr Ile Met
            420                 425                 430

Leu Val Lys Arg Gln Val Pro Leu Asp Cys Val Leu Tyr Arg Tyr Gly
        435                 440                 445

Ser Phe Ser Leu Ala Leu Asp Ile Val Gln Gly Ile Glu Ser Ala Glu
    450                 455                 460

Ile Leu Gln Ala Val Pro Phe Ser Glu Gly Asp Ala Phe Glu Leu Thr
465                 470                 475                 480

Val Ser Cys Gln Gly Gly Leu Pro Lys Glu Ala Cys Met Asp Ile Ser
                485                 490                 495

Ser Pro Gly Cys Gln Pro Pro Ala Gln Arg Leu Cys Gln Ser Val Pro
            500                 505                 510

Pro Ser Pro Asp Cys Gln Leu Val Leu His Gln Val Leu Lys Gly Gly
        515                 520                 525

Ser Gly Thr Tyr Cys Leu Asn Val Ser Leu Ala Asp Ala Asn Ser Leu
    530                 535                 540

Ala Val Ala Ser Thr Gln Leu Val Val Pro Gly Gln Asp Gly Gly Leu
545                 550                 555                 560

Gly Gln Ala Pro Leu Leu Val Gly Ile Leu Leu Val Leu Val Ala Val
                565                 570                 575

Val Leu Ala Ser Leu Ile Leu Gly Ile Asp Leu Arg Ser Arg Ala Gln
            580                 585                 590

Phe Pro Lys Cys His Met Val Ala Leu Thr Ala Ala Pro Ala Ser Gly
        595                 600                 605

Leu Arg Ala Arg Gly Leu Gly Glu Asn Ser Pro Leu Leu Ser Gly Gln
    610                 615                 620

Gln Val
625

<210> 3
<211> 2131
<212> DNA
<213> Homo sapiens

<220>
<221> CDS
<222> (12)..(2018)

<400> 3
ggaagaacac a atg gat ctg gtg cta aaa aga tgc ctt ctt cat ttg gct   50
gtg ata ggt gct ttg ctg gct gtg ggg gct aca aaa gta ccc aga aac   98
cag gac tgg ctt ggt gtc tca agg caa ctc aga acc aaa gcc tgg aac   146
agg cag ctg tat cca gag tgg aca gaa gcc cag aga ctt gac tgc tgg   194
aga ggt ggt caa gtg tcc ctc aag gtc agt aat gat ggg cct aca ctg   242
att ggt gca aat gcc tcc ttc tct att gcc ttg aac ttc cct gga agc   290
caa aag gta ttg cca gat ggg cag gtt atc tgg gtc aac aat acc atc   338
atc aat ggg agc cag gtg tgg gga gga cag cca gtg tat ccc cag gaa   386
act gac gat gcc tgc atc ttc cct gat ggt gga cct tgc cca tct ggc     434
tct tgg tct cag aag aga agc ttt gtt tat gtc tgg aag acc tgg ggc     482
caa tac tgg caa gtt cta ggg ggc cca gtg tct ggg ctg agc att ggg     530
aca ggc agg gca atg ctg ggc aca cac acc atg gaa gtg act gtc tac     578
cat cgc cgg gga tcc cgg agc tat gtg cct ctt gct cat tcc agc tca     626
gcc ttc acc att act gac cag gtg cct ttc tcc gtg agc gtg tcc cag     674
ttg cgg gcc ttg gat gga ggg aac aag cac ttc ctg aga aat cag cct     722
ctg acc ttt gcc ctc cag ctc cat gac cct agt ggc tat ctg gct gaa     770
gct gac ctc tcc tac acc tgg gac ttt gga gac agt agt gga acc ctg     818
atc tct cgg gca cct gtg gtc act cat act tac ctg gag cct ggc cca     866
gtc act gcc cag gtg gtc ctg cag gct gcc att cct ctc acc tcc tgt     914
ggc tcc tcc cca gtt cca ggc acc aca gat ggg cac agg cca act gca     962
gag gcc cct aac acc aca gct ggc caa gtg cct act aca gaa gtt gtg     1010
ggt act aca cct ggt cag gcg cca act gca gag ccc tct gga acc aca     1058
tct gtg cag gtg cca acc act gaa gtc ata agc act gca cct gtg cag 1106
atg cca act gca gag agc aca ggt atg aca cct gag aag gtg cca gtt 1154
tca gag gtc atg ggt acc aca ctg gca gag atg tca act cca gag gct 1202
aca ggt atg aca cct gca gag gta tca att gtg gtg ctt tct gga acc 1250
aca gct gca cag gta aca act aca gag tgg gtg gag acc aca gct aga 1298
gag cta cct atc cct gag cct gaa ggt cca gat gcc agc tca atc atg 1346
tct acg gaa agt att aca ggt tcc ctg ggc ccc ctg ctg gat ggt aca 1394
gcc acc tta agg ctg gtg aag aga caa gtc ccc ctg gat tgt gtt ctg 1442
tat cga tat ggt tcc ttt tcc gtc acc ctg gac att gtc cag ggt att 1490
gaa agt gcc gag atc ctg cag gct gtg ccg tcc ggt gag ggg gat gca 1538
ttt gag ctg act gtg tcc tgc caa ggc ggg ctg ccc aag gaa gcc tgc 1586
atg gag atc tca tcg cca ggg tgc cag ccc cct gcc cag cgg ctg tgc 1634
cag cct gtg cta ccc agc cca gcc tgc cag ctg gtt ctg cac cag ata 1682
ctg aag ggt ggc tcg ggg aca tac tgc ctc aat gtg tct ctg gct gat 1730
acc aac agc ctg gca gtg gtc agc acc cag ctt atc atg cct gtg cct 1778
ggg att ctt ctc aca ggt caa gaa gca ggc ctt ggg cag gtt cgg ctg 1826
atc gtg ggc atc ttg ctg gtg ttg atg gct gtg gtc ctt gca tct ctg 1874
ata tat agg cgc aga ctt atg aag caa gac ttc tcc gta ccc cag ttg 1922
cca cat agc agc agt cac tgg ctg cgt cta ccc cgc atc ttc tgc tct 1970
tgt ccc att ggt gag aat agc ccc ctc ctc agt ggg cag cag gtc tga 2018
gtactctcat atgatgctgt gattttcctg gagttgacag aaacacctat atttccccca 2078
gtcttccctg ggagactact attaactgaa ataaatactc agagcctgaa aaa 2131

<210> 4
<211> 668
<212> PRT
<213> Homo sapiens

<400> 4

<210> 5
<211> 9
<212> PRT
<213> Homo sapiens

<400> 5
Lys Thr Trp Gly Gln Tyr Trp Gln Val
1               5

<210> 6
<211> 9
<212> PRT
<213> Homo sapiens

<400> 6

Ile Thr Asp Gln Val Pro Phe Ser Val
1               5

<210> 7
<211> 10
<212> PRT
<213> Homo sapiens

<400> 7
Val Leu Tyr Arg Tyr Gly Ser Phe Ser Val
1               5                   10

<210> 8
<211> 9
<212> PRT
<213> Homo sapiens

<400> 8
Lys Thr Trp Gly Lys Tyr Trp Gln Val
1               5

<210> 9
<211> 10
<212> PRT
<213> Homo sapiens

<400> 9
Phe Leu Thr Pro Lys Lys Leu Gln Cys Val
1               5                   10

<210> 10
<211> 9
<212> PRT
<213> Homo sapiens

<400> 10
Lys Leu Gln Cys Val Asp Leu His Val
1               5

<210> 11
<211> 10
<212> PRT
<213> Homo sapiens

<400> 11
Val Ile Ser Asn Asp Val Cys Ala Gln Val
1               5                   10

<210> 12
<211> 10
<212> PRT
<213> Homo sapiens

<400> 12
Gln Val His Pro Gln Lys Thr Val Thr Lys
1               5                   10

<210> 13
<211> 9
<212> PRT
<213> Homo sapiens

<400> 13
His Leu Phe Gly Tyr Ser Trp Tyr Lys
1               5

## Description

The present invention relates to a pharmaceutical or vaccine composition comprising a nucleic acid molecule encoding a tumor-associated antigen and at least one peptide comprising a region corresponding to a putative cytotoxic T cell, helper T cell or B cell epitope of said tumor-associated antigen and/or cells pulsed with such peptide(s) optionally in combination with a pharmaceutically acceptable carrier. Such a pharmaceutical or vaccine composition can be used for the treatment of cancer or for the vaccination against cancer.

Cancer is one of the major causes of death and therapies include nonspecific chemo- and radiation therapy which normally do not result in cure. Until today no approved cancer vaccine is known. Several experimental vaccines are being tested mainly as compassionate trials. An exception is a humanized monoclonal antibody, Herceptin, against a small portion of human mammary carcinoma.
For a specific immunotherapy against cancer, various vaccines have been developed to deliver tumor-associated antigens to the immune system such that immune cells recognize the antigen as foreign and destroy any cells bearing this antigen. Recent research has focused on the presentation of antigenic peptides bound to major histocompatibility complex (MHC) molecules on the cell surface of antigen-presenting cells (APCs) to T cells. One of the best-characterized melanoma-associated antigens is the pmel17/gp100. The gp100 gene product, a glycoprotein of 100 kDa, is present in the filamentous matrix of melanosomes and is involved in the late stages of melanin synthesis. Expression of gp100 is found in melanomas, normal melanocytes, substantia nigra and retina, but not in other tissues or non-melanoma tumors (Rosenberg, Immunol. Today 18 (1997), 175-182). Thus, gp100 appears to be a normal melanocyte differentiation antigen.
The recent identifications of peptide sequences from such melanoma-associated antigens that are immunogenic in humans and that are recognized by CD8⁺ T cells *in vivo* has led to the construction of new specific melanoma vaccines. Phase I immunization trials using gp100 peptides with or without co-administration of recombinant cytokines such as IL-2, IL-12 or GM-CSF led to an enhancement of antigen-specific CTL responses (Kawakami et al., J. Immunther. 21 (1998), 237-246). Another work demonstrated an additive effect of GM-CSF in combination with gp100 as antitumor DNA vaccine in a syngeneic melanoma mouse model (Nawrath et al., Adv. Exp. Med. Biol. 451 (1998), 305-310; Nawrath et al., Leukemia 13 (1999), 48-51). Moreover, intradermal or intramuscular injection of gp100 antigen-encoding plasmid DNA induced CTL-mediated tumor protection in a mouse model. However, this tumor protection has only been achieved previously when the tumor cells, used for the challenge, were transfected with the xenoantigen used for vaccination. The DNA xenoimmunization failed to protect mice against challenge with tumor cells expressing mouse gp100 (Schreurs et al., Cancer Res. 58 (1998), 2509-2514).
DNA vaccine technology offers considerable promise for the improvement of existing immunization strategies (for reviews: Ulmer et al., Curr. Opin. Immunol. 8 (1996), 531-536; Tuting et al., J. Invest. Dermatol. 111 (1998), 183-188; Kumar and Sercarz, Nat. Med. 2 (1996), 857-859). It involves the simple injection of naked plasmid DNA vectors that encode antigens and/or cytokines under the control of an eukaryotic promoter, and results in strong and sustained humoral and cell-mediated immune responses. These responses can be protective and therapeutic against infectious and neoplastic diseases which could not be successfully treated with conventional immunization strategies so far. Direct injection of DNA into an organism may mimic a normal infection since the antigen is produced by the host and therefore may be processed and post-translationally modified identically to the native antigens. Endogenous protein synthesis leads to both, presentation of the foreign antigen by MHC class I molecules as well as uptake of soluble protein fragments by APCs and presentation by MHC class II molecules, such that both arms of the immune system are induced (Nawrath et al., (1999), loc. cit.; Kumar and Sercarz, loc. cit.; Maecker et al., J. Immunol. 161 (1998), 6532-6536).
The induction of a strong and long-lasting immunity characterized by both a humoral and cell-mediated immune response is one of the most important considerations for developing effective antitumor vaccines.

The technical problem underlying the present invention is the provision of means and methods allowing an efficient treatment and/or prevention of cancer such as melanoma.

This problem has been solved by the provision of the embodiments as characterized in the claims.

Accordingly, the present invention relates to a pharmaceutical or vaccine composition comprising a nucleic acid molecule encoding a tumor-associated antigen and at least one peptide comprising a region corresponding to a putative cytotoxic T cell, helper T cell or B cell epitope of said tumor-associated antigen and/or cells pulsed with such peptide(s), optionally in combination with a pharmaceutically acceptable carrier and/or adjuvant.

It has been surprisingly found that applying a combination of DNA encoding a tumor-associated antigen together with peptides derived from said tumor-associated antigen or cells pulsed with such peptides effectively protected mice from developing cancer, in that specific case melanoma, whereas the application of DNA or peptides/cells alone did not have this effect.

In the following the term "pharmaceutical composition" refers to both, pharmaceutical compositions as well as to vaccine compositions.

The term "tumor-associated antigen" in this context refers to a structure which is preferably presented by tumor cells and thus allows a distinction between tumor cells and non-tumor cells. Tumor associated antigens are proteins expressed inside or on the surface of tumor cells which are putative targets for immune responses. They often differ from normal cellular counterparts by mutations, deletions, different levels of expression, changes in secondary modifications or expression in other stages of development. The proteins are preferably expressed on the cellular surface and, in addition, presented as processed peptides on the tumor cell surface by MHC class I molecules. Examples for tumor-associated antigens are:
CA125
CA19-9
CA15-3
D97
gp100
CD20
CD21
TAG-72
EGF receptor
Epithelial cell adhesion molecule (Ep-CAM)
Carcino embryonic antigen (CEA)
Prostate specific antigen (PSA)
Her2/Neu receptor
tyrosinase
MAGE 1
MAGE 3
MART
BAGE
TRP-1
CA 50
CA 72-4
MUC 1
NSE (neuron specific enolase)
α-fetoprotein (AFP)
SSC (squamous cell carcinoma antigen)
BRCA-1
BRCA-2 and
hCG.

In a preferred embodiment the tumor-associated antigen is gp100. Therefore, the present invention preferably relates to a pharmaceutical or vaccine composition comprising a nucleic acid molecule encoding a gp100 protein and at least one peptide comprising a region corresponding to a putative cytotoxic T cell epitope of a gp100 protein and/or cells pulsed with such peptide(s), optionally in combination with a pharmaceutically acceptable carrier and/or an adjuvant. Such a composition can be effectively used to treat or prevent melanoma.

In the context of the present invention the term "gp100 protein" relates to a melanoma-associated antigen that is expressed in melanocytes and highly tumorigenic B16 melanoma cells. This protein has been described, e.g., in Wagner et al. (Laboratory Investigation 73 (1995), 229-235). The human protein is 668 amino acids long. However, there is also a variant, i.e. a splice variant, which lacks 7 amino acids (588 to 594). The term "gp100" therefore also covers splice variants of a gp100 protein. gp100 is described as being present in cells of the melanoma/melanocytic lineage, including junctional and blue nevocellular nevi, dysplastic nevocellular nevi, horizontal and vertical growth phase melanoma, metastatic melanoma and fetal melanocytes. In particular, it is present in the filamentous matrix of melanosomes and is involved in the late stages of melanin synthesis. It has been described that it is also expressed in substantia nigra and retina but not in non-melanoma tumors (Rosenberg, loc. cit.). Furthermore, it is described as being detected by the monoclonal antibody HMB-45. The protein is preferably a glycoprotein and more preferably has a molecular weight of about 100 kDa when determined by sodium dodecyl (SDS) sulphate-polyacrylamide gel electrophoresis (PAGE).
The gp100 protein is preferably a protein encoded by a nucleic acid molecule encoding a gp100 protein selected from the group consisting of
(a) nucleic acid molecules encoding a protein which comprises the amino acid sequence indicated in SEQ ID NO:2 or in SEQ ID NO:4;
(b) nucleic acid molecules comprising the nucleotide sequence of the coding region indicated in SEQ ID NO: 1 or in SEQ ID NO:3;
(c) nucleic acid molecules the complementary strand of which hybridizes to a nucleic acid molecule as defined in (a) or (b) and which encode a gp100 protein; and
(d) nucleic acid molecules, the nucleotide sequence of which deviates because of the degeneracy of the genetic code from the sequence of the nucleic acid molecules as defined in (b) or (c).

In this context the term "hybridization" means hybridization under conventional hybridization conditions, preferably under stringent conditions, as for instance described in Sambrook et al., Molecular Cloning, A Laboratory Manual, 2^{nd} edition (1989) Cold Spring Harbor Laboratory Press, Cold Spring Harbor, NY. In an especially preferred embodiment the term "hybridization" means that hybridization occurs under the following conditions:
- Hybridization buffer:: 2 x SSC; 10 x Denhardt solution (Fikoll 400 + PEG + BSA; ratio 1:1:1); 0.1% SDS; 5 mM EDTA; 50 mM Na₂HPO_{4;}
250 µg/ml of herring sperm DNA; 50 µg/ml of tRNA;
or
0.25 M of sodium phosphate buffer, pH 7.2;
1 mM EDTA
7% SDS
- Hybridization temperature T: = 60°C
- Washing buffer:: 2 x SSC; 0.1 % SDS
- Washing temperature T: = 60°C.

Nucleic acid molecules which hybridize with the above mentioned nucleic acid molecules can, in principle, encode a gp100 protein from any organism expressing such a protein or can encode modified versions thereof.
Nucleic acid molecules which hybridize with the above-mentioned molecules can for instance be isolated from genomic libraries or cDNA libraries. Preferably, such molecules are from animal origin, particularly preferred from human origin. Alternatively, they can be prepared by genetic engineering or chemical synthesis.
Such nucleic acid molecules may be identified and isolated by using the above-mentioned molecules or parts of these molecules or reverse complements of these molecules, for instance by hybridization according to standard methods (see for instance Sambrook et al., 1989, Molecular Cloning. A Laboratory Manual, 2^{nd} edition, Cold Spring Harbor Laboratory Press, Cold Spring Harbor, NY).
Nucleic acid molecules comprising the same or substantially the same nucleotide sequence as indicated in SEQ ID NO:1 or in SEQ ID NO:3 or parts thereof can, for instance, be used as hybridization probes. The fragments used as hybridization probes can also be synthetic fragments which are prepared by usual synthesis techniques, and the sequence of which is substantially identical with that of a nucleic acid molecule as shown in SEQ ID NO:1 or in SEQ ID NO:3.
The molecules hybridizing with the above-mentioned nucleic acid molecules also comprise fragments, derivatives and allelic variants of the above-described nucleic acid molecules encoding a gp100 protein. Herein, fragments are understood to mean parts of the nucleic acid molecules which are long enough to encode the described protein, preferably showing the biological activity of a gp100 protein described above. In this context, the term derivative means that the sequences of these molecules differ from the sequences of the above-described nucleic acid molecules in one or more positions and show a high degree of homology to these sequences. In this context, homology means a sequence identity of at least 40%, in particular an identity of at least 60%, preferably of more than 65%, even more preferably of at least 70%, in particular of at least 80%, more preferably of at least 90% and particularly preferred of more than 95%. Deviations from the above-described nucleic acid molecules may have been produced, e.g., by deletion, substitution, insertion andlor recombination.
Preferably, the degree of homology is determined by comparing the respective sequence with the nucleotide sequence of the coding region of SEQ ID No: 1 or of SEQ ID NO:3. When the sequences which are compared do not have the same length, the degree of homology preferably refers to the percentage of nucleotide residues in the shorter sequence which are identical to nucleotide residues in the longer sequence. The degree of homology can be determined conventionally using known computer programs such as the DNASTAR program with the Clustalw analysis. This program can be obtained from DNASTAR, Inc., 1228 South Park Street, Madison, WI 53715 or from DNASTAR, Ltd., Abacus House, West Ealing, London W13 OAS UK (support@dnastar.com) and is accessible at the server of the EMBL outstation.
When using the Clustal analysis method to determine whether a particular sequence is, for instance, 80% identical to a reference sequence the settings are preferably as follows: PAIRGAP: 0.05; MATRIX: blosum; GAP OPEN: 10; END GAPS: 10; GAP EXTENSION: 0.05; GAP DISTANCE: 0.05; KTUP: 1; WINDOW LENGTH: 0; TOPDIAG: 1.
Furthermore, homology means preferably that the encoded protein displays a sequence identity of at least 40%, more preferably of at least 50%, even more preferably of at least 60%, in particular of at least 65%, particularly preferred of at least 70%, especially preferred of at least 80% and even more preferred of at least 90% to the amino acid sequence depicted under SEQ ID NO: 2 or under SEQ ID NO:4.
Preferably, sequences hybridizing to a nucleic acid molecule according to the invention comprise a region of homology of at least 90%, preferably of at least 93%, more preferably of at least 95%, still more preferably of at least 98% and particularly preferred of at least 99% identity to an above-described nucleic acid molecule, wherein this region of homology has a length of at least 1000 nucleotides, more preferably of at least 1250 nucleotides, even more preferably of at least 1500 nucleotides, particularly preferred of at least 1750 nucleotides and most preferably of at least 2000 nucleotides.
Homology, moreover, means that there is a functional and/or structural equivalence between the corresponding nucleic acid molecules or proteins encoded thereby. Nucleic acid molecules which are homologous to the above-described molecules and represent derivatives of these molecules are normally variations of these molecules which represent modifications having the same biological function. They may be either naturally occurring variations, for instance sequences from other ecotypes, varieties, species, etc., or mutations, and said mutations may have formed naturally or may have been produced by deliberate mutagenesis. Furthermore, the variations may be synthetically produced sequences. The allelic variants may be naturally occurring variants or synthetically produced variants or variants produced by recombinant DNA techniques.
The proteins encoded by the different variants of the above-mentioned nucleic acid molecules possess certain characteristics they have in common. These include for instance biological activity, molecular weight, immunological reactivity, conformation, etc., and physical properties, such as for instance the migration behavior in gel electrophoreses, chromatographic behavior, sedimentation coefficients, solubility, spectroscopic properties, stability, pH optimum, temperature optimum etc.

The nucleic acid molecule encoding a tumor-associated antigen does not necessarily have to encode the full-length protein. It should, however, be long enough to encode a protein which is capable of eliciting an effective immune response or may comprise those sequences encoding an amino acid sequence which can elicit an effective immune response. Thus, it may e.g. code for one or more individual putative T cell epitopes optionally linked in tandem either directly or separated by spacer sequences. Molecules comprising several epitopes in tandem separated by spacer sequences are also called polyepitopes.

The nucleic acid molecule contained in the pharmaceutical composition according to the invention can be any kind of nucleic acid molecule, e.g. RNA or DNA or RNA/DNA hybrids. Preferably it is a DNA molecule. Such DNA molecules may be, e.g., genomic or cDNA molecules. They may be isolated from natural sources by means known to the person skilled in the art, e.g. PCR amplification, or synthesized in vitro or by chemical synthesis.
The nucleic acid molecule may be a molecule comprising a sequence encoding a tumor-associated antigen sequence wherein said sequence does occur in nature. Alternatively, the tumor-associated antigen encoding sequence may be modified, e.g. by deletion, insertion, addition or substitution of one or more nucleotides.

In a preferred embodiment the nucleic acid molecule contained in the pharmaceutical composition according to the invention is a cDNA molecule, most preferably a cDNA molecule encoding a mouse or human gp100 protein and most preferably a cDNA molecule encoding the amino acid sequence as depicted under SEQ ID NO: 2 or under SEQ ID NO: 4.

In another preferred embodiment the nucleic acid molecule contained in the pharmaceutical composition according to the invention encodes a tumor-associated antigen which is heterologous with respect to the species to which the individual belongs to which the pharmaceutical composition shall be applied. Such a pharmaceutical composition can in particular be used for xenoimmunization. This means that immunization is carried out with an antigen which differs slightly from the antigen as it naturally occurs in the individual which is being vaccinated. Therefore, in a preferred embodiment the tumor-associated antigen encoded by the nucleic acid molecule differs at at least one position, preferably at at least two positions, more preferably at at least 5 positions, even more preferably at at least 10 positions of its amino acid sequence from the corresponding tumor-associated antigen which naturally occurs in the species to which the individual belongs to which the pharmaceutical composition shall be administered. It could be shown that such a xenoimmunization results in a much better prevention or treatment of cancer, in particular of melanoma.

The nucleic acid molecule contained in the pharmaceutical composition according to the invention, apart from the nucleotide sequence encoding a tumor-associated antigen, can also comprise regulatory elements allowing expression of the coding sequence in a eukaryotic host cell. Such regulatory elements comprise for example promoters, enhancers and transcription terminators.

As a promoter any promoter can be used which ensures expression in eukaryotic host cells, in particular in those cells which should be targeted by the pharmaceutical composition. Promoters can be used which allow for a constitutive expression or for inducible expression. Various promoters possessing these properties are described in detail in the literature. Suitable promoters/enhancers are in particular viral, prokaryotic, e.g. bacterial or eukaryotic promoterslenhancers, e.g., the adenoviral E1A promoter, the MLP promoter, the phosphoglycero kinase promoter (Adra et al., Gene 60 (1987), 65-74; Hitzman et al., Science 219 (1983), 620-625), the metallothioneine promoter (Mclvor et al., Mol. Cell. Biol. 7 (1987), 838-848), the CTFR promoter, the early SV40 promoter, the RSV LTR promoter, the α-1 antitrypsin promoter, the β-actin promoter (Tabin et al., Mol. Cell. Biol. 2 (1982), 426-436), the CEA promoter (Schrewe et al., Mol. Cell. Biol. 10 (1990), 2738-2748), the MUC-1 promoter (Chen et al., J. Clin. Invest. 96 (1995), 2775-2782), the ErbB-2 promoter (Harris et al., Gene Therapy 1 (1994), 170-175), the MAGE1 promoter/enhancer (De Smet et al., Immunogenetices 42 (1995), 282-290), the tyrosinase promoter/enhancer (Bentley et al., Mol. Cell. Biol. 14 (1994), 7996-8006), the tyrosinase-related protein 1 promoter/enhancer (Lowings et al., Mol. Cell. Biol. 12 (1992), 3653-2662), and the MART-1/Melan-A promoter/enhancer. (Butterfield et al., Gene 191 (1997), 129-134).
Preferably, the CMV early promoter/enhancer region is used.
Transcription terminators which can be used are also known to the person skilled in the art and are described in the literature. Preferably, the terminator of the bovine growth hormone (BGH) is used.
Moreover, the nucleic acid molecule may also comprise sequences enhancing translation, such as plant viral translational enhancers, e.g. the tobacco mosaic virus translational enhancer (Turner and Foster, Mol. Biotechnol. 3 (1995), 225-236) and animal RNA virus translational enhancers, e.g. Sindbis virus translational enhancer (Frolov and Schlesinger, J. Virol. 70 (1996), 1182-1190).
It may also comprise further elements such as intron(s), secretion signals, nuclear localization signals, IRES, tripartite leader sequences etc.

The nucleic acid molecule contained in the pharmaceutical composition according to the invention may be, e.g., in the form of an expression vector. Such vectors generally contain a selection marker gene and a replication-origin ensuring replication, e.g., in a host cell used for propagation of the expression vector.

Furthermore, they generally contain a multiple cloning site containing various restriction sites allowing integration of an expression cassette. The nucleic acid molecule may comprise one or more sequences encoding a tumor-associated antigen, controlled by the same or different regulatory elements.

The nucleic acid molecule contained in the pharmaceutical composition may be in any possible form which is suitable for its administration to a patient. It may, for example, be single stranded and/or double stranded, linear or circular, or complexed to other components such as proteins or lipids. In a preferred embodiment it is "naked", i.e. not complexed to other components. The nucleic acid may be in form of a double stranded, linear DNA, linear single stranded RNA or preferably in form of a double stranded closed circular DNA plasmid.

The nucleic acid molecule contained in the pharmaceutical composition can be associated with a vehicle facilitating or improving transfection, such as cationic lipids, cationic polymers, polypeptides or viral particles. Viral particles include in particular adenoviral and retroviral particles. Such particles have already been used in a large number of gene delivery applications in gene therapy or vaccination.
Other compounds facilitating or improving transfection are, e.g., polyethylene imine or polypropylene, polylysine, polyamidoamine or lipids such as DOTMA, DOGS, Transfectam™ (Behr et al., Proc. Natl. Acad. Sci. USA 86 (1989), 6982-6986), DMRIE, DORIE (Felgner et al., Methods 5 (1993), 67-75), DC-Chol (Gao and Huang, BBRC 179 (1991), 280-285), DOTAP™, Lipofectamine™, microparticles, e.g. poly(lactide-coglycolide) (PLG) microparticles (Chen et al., J. Virol. 72 (1998), 5757-5761) or virosomes, e.g. infuenza virosomes (Cusi et al., Virology 277 (2000), 111-118).

The pharmaceutical composition according to the invention may also comprise a pharmaceutical acceptable carrier and/or an adjuvant normally used in the preparation of pharmaceutical or vaccine formulations, in particular for use in the therapeutic treatment or vaccination of humans or animals. The pharmaceutically acceptable carrier or adjuvant is preferably a carrier or adjuvant which is injectable. Preferably it is isotonic, hypotonic or weakly hypotonic and has a relatively low ionic strength. An example is a sucrose solution. Other examples are water, isotonic saline solutions, preferably buffered at a physiological pH (e.g. phosphate buffered saline), mannitol, glycerol containing e.g. human serum albumin.
The composition may be manufactured according to convential methods known to the person skilled in the art, in particular in such a way that it is suitable for local, systemic, oral, rectal or topical administration. Possible routes of administration include but are not limited to intragastric, subcutaneous, intravenous, intraarterial, intraperitoneal, aerosol, instillation, inhalation, intramuscular, intranasal, intratracheal, intrapulmonary, intratumoral or intracardiac.
The administration route and dosage generally vary depending on various parameters, such as, the age, weight, health condition, stage to which the disease state has progressed, the need for prevention or therapy, etc., of the individual to be treated, the disease state, etc.

The pharmaceutical composition may also comprise buffering solutions, stabilizing agents, proteins to protect against nucleases or preservatives adapted to the administration route.

The pharmaceutical composition according to the invention also comprises at least one peptide comprising a region corresponding to a putative cytotoxic T cell, helper T cell or B cell epitope of a tumor-associated antigen, preferably of the tumor-associated antigen which is encoded by the nucleic acid molecule contained in the pharmaceutical composition according to the invention. In this context cytotoxic T cell epitopes are understood to be short antigenic peptides (preferably nonamers or decamers) derived from self or foreign proteins that are processed intracellularly, bound and presented by MHC class I molecules at the surface of cells. These complexes are recognized by T cell receptors on CD8 positive T cells in conjunction with the CD8 molecule. Putative cytotoxic T cell epitopes of a given protein can be predicted using specific computer programs that calculate the scores of every possible peptide from a protein sequence. The prediction is based on published motifs (pool sequencing, natural ligands) and takes into consideration the amino acids in the anchor and auxiliary anchor positions, as well as other frequent amino acids (Rammensee et al., Immunogenetics 50 (1999), 213-219). Putative T cell epitopes can, e.g., be identified using the HLA-binding prediction (http://bimas.cit.nih.gov/molbio/hla_bind/; Parker et al., J. Immunol. 152 (1994), 163). Furthermore, helper T cell epitopes are understood to be short antigenic peptides (preferably 13 to 18 mers) derived from self or foreign proteins that are processed intracellularly, bound and presented by MHC class II molecules at the surface of cells. These complexes are recognized by T cell receptors on CD4 positive T cells in conjunction with the CD4 molecule. The putative helper T cell epitopes for MHC class II molecules comprise all peptides of the amino acid sequence of a given tumor associated antigen that reach 50% of the maximal score using the SYFPEITHI epitope prediction algorithm (http://134.2.96.221/scripts/MHCServer.dll/home.htm). The scoring system evaluates every amino acid within a given peptide. Individual amino acids may be given the arbitrary value 1 for amino acids that are only slightly preferred in the respective position, optimal anchor residues are given the value 15; any value between these two is possible. Negative values are also possible for amino acids which are disadvantageous for the peptide's binding capacity at a certain sequence position. The allocation of values is based on the frequency of the respective amino acid in natural ligands, T-cell epitopes, or binding peptides (HG Rammensee, J Bachmann, NPN Emmerich, OA Bachor and S Stevanovic (1999) SYFPEITHI: database for MHC ligands and peptide motifs. Immunogenetics 50: 213-219).
B cell epitopes can consist of short peptide sequences (e.g. 6 amino acids) or carbohydrates. Peptide derived B cell eptiopes can contain sequential or nonsequential, conformational epitopes. B cell epitopes in native proteins generally are hydrophilic amino acids on the protein surface that are topographically accessible to membrane bound or free antibody.

Putative T cell epitopes are known for many tumor associated antigens. For example the following cytotoxic T cell epitopes have been characterized for PSA and CEA (Correale et al., J. Immunol. 161 (1998), 3186-3194):

| | | |
|---|---|---|
| PSA-1 | aa141-170 | FLTPKKLQCV (SEQ ID NO: 9) |
| PSA-2 | aa146-154 | KLQCVDLHV (SEQ ID NO: 10) |
| PSA-3 | aa154-163 | VISNDVCAQV (SEQ ID NO: 11) |
| PSA-9 | aa162-170 | QVHPQKTVTK (SEQ ID NO: 12) |
| CEA27 | aa27-35 | HLFGYSWYK (SEQ ID NO: 13) |

In a preferred embodiment the tumor-associated antigen is a gp100 protein and such a peptide comprises an amino acid sequence corresponding to positions 280 to 289, 52 to 61, 626 to 635, 609 to 618, 154 to 163, 604 to 613, 18 to 27, 178 to 187, 162 to 171, 608 to 617, 290 to 299, 69 to 78, 584 to 593, 613 to 622, 624 to 633, 478 to 487, 468 to 477, 6 to 15, 603 to 612, 146 to 155, 222 to 231, 61 to 70, 629 to 638, 625 to 634, 190 to 199, 469 to 478, 550 to 559, 313 to 322, 175 to 184, 194 to 203, 645 to 654, 271 to 280, 537 to 546, 45 to 54, 233 to 242, 242 to 251 or 29 to 38 of the amino acid sequence of the human gp100 protein (SEQ ID NO:4) or an amino acid sequence of a peptide of a gp100 protein from another species corresponding to the above-mentioned regions of the human gp100 protein. Examples of putative cytotoxic T cell epitopes of gp100 proteins from other species are, e.g., peptides comprising an amino acid sequence corresponding to position 427 to 436, 569 to 578, 47 to 56, 32 to 41, 154 to 163, 291 to 300, 178 to 187, 13 to 22, 613 to 622, 568 to 577, 268 to 277, 559 to 568, 70 to 79, 243 to 252, 290 to 299, 445 to 454, 6 to 15, 5 to 14 or 435 to 444 of the amino acid sequence of the mouse gp100 protein (SEQ ID NO:2).
Other examples are peptides comprising an amino acid sequence corresponding to positions 409 to 418, 399 to 408, 218 to 227, 82 to 91, 272 to 281, 426 to 435, 52 to 61, 413 to 422, 99 to 108, 51 to 60, 285 to 294, 275 to 284, 425 to 434, 236 to 245, 424 to 433, 276 to 285, 256 to 265, 357 to 366, 80 to 89, 244 to 253, 447 to 456, 216 to 225, 453 to 462, 36 to 45, 254 to 263, 29 to 38, 44 to 53, 234 to 243 or 246 to 255 of the horse gp100 protein sequence as published in Rieder et al. (Sequence submission to GenBank Database Accession No. AF076780). In another preferred embodiment the peptide comprises an amino acid sequence which shows at least 55%, preferably at least 70%, more preferably at least 80% and even more preferably at least 90% sequence identity to one of the above mentioned peptide sequences.

In a particularly preferred embodiment the at least one peptide contained in the pharmaceutical composition according to the invention comprises one or more of the following gp100 amino acid sequences:
(i) KTWGQYWQV (SEQ ID NO:5);
(ii) ITDQVPFSV (SEQ ID NO:6);
(iii) VLYRYGSFSV (SEQ ID NO:7); and
(iv) KTWGKYWQV (SEQ ID NO:8).

In a preferred embodiment the peptide comprises an amino acid sequence of a tumor-associated antigen which is heterologous with respect to the species to which the individual belongs to which the pharmaceutical composition shall be administered (xenoimmunization). Preferably, the amino acid sequence of the peptide differs at at least one position from the corresponding amino acid sequence of the tumor-associated antigen naturally occurring in the species to which the individual to be treated belongs, more preferably at at least two positions, even more preferably at at least three positions and most preferably at at least four positions.

The peptide contained in the pharmaceutical composition preferably has a length of at least 4 amino acids, more preferably of at least 6 amino acids, even more preferably of at least 9 amino acids and particularly preferred of at least 12 amino acids. The peptide furthermore preferably is not longer than 100 amino acids, more preferably not longer than 50 amino acids, even more preferably not longer than 30 amino acids and particularly preferred not longer than 20 amino acids.
Such peptides can be prepared according to methods known to the person skilled in the art, e.g. by chemical synthesis. The peptides may also comprises modifications such as covalent addition of Pam3 Cys as an adjuvant. Many of the outer membrane proteins of gram-negative bacteria are both lipid-modified and very immunogenic. Because of the apparent correlation between covalent lipid linkage and immunogenicity, tripalmitoyl-S-glycerol cysteine (Pam3 Cys), a lipid common to bacterial membrane proteins, can be coupled to peptides representing either B cell or cytotoxic T cell epitopes. The peptides can for example be administered by the following routes: subcutaneous, intradermal or intralymphatic.

Instead of being directly administered to an individual, the peptides contained in the pharmaceutical composition according to the invention may also be used for pulsing cells ex vivo as described below. These cells may then be administered to an individual in parallel, simultaneously, before or after administration of the nucleic acid molecule encoding a tumor-associated antigen.

The pharmaceutical composition according to the invention may also comprise, instead of or in addition to the above-mentioned peptide(s), cells which were previously pulsed with the above-described peptide(s). The technique of pulsing cells with different types of compounds, in particular proteins or peptides, is known to the person skilled in the art and is described, for example, in Nestle et al. (Nat. Med. 4 (1998), 328-332). In brief, cells are transferred into a suitable medium and incubated in vitro for an appropriate time with the compound. Before injection the cells are washed, e.g. three times in sterile PBS, and resuspended in a suitable volume of, e.g. PBS. Antigen-loaded cells are administered immediately, generally in an inguinal lymph node (Nestle et al., loc. cit.). The cells are preferably dendritic cells (DCs).

In a preferred embodiment the cells used for peptide pulsing are of the same species as the individual to whom the pharmaceutical composition should be applied. In a particularly preferred embodiment the cells are derived from the same individual to whom the pharmaceutical composition should be applied.

The different compounds of the pharmaceutical composition according to the invention (i.e. nucleic acid molecule, polypeptide(s) and/or peptide-pulsed cells) may be present in the pharmaceutical composition in one compartment, e.g. in a form such that they can be applied together in one physical entity.
However, in a preferred embodiment the different components are present in the pharmaceutical composition in different compartments, i.e. they constitute separate parts of the pharmaceutical composition. This allows to administer the different components separately, e.g. via different routes of administrations and/or at different points in time. Thus, the pharmaceutical composition is preferably designed as a kit comprising separate compartments for the nucleic acid molecule, the peptide(s) and/or the peptide-pulsed cells.

Administration of the pharmaceutical composition according to the invention may be as a single dose or as repeated doses, once or several times after a certain period of time. The pharmaceutical composition according to the invention is furthermore, preferably designed for repeated administrations of one or more of its components, i.e. nucleic acid molecule, peptide(s) and/or cells). Repeated administration may allow to reduce the dose of the respective component to be administered. When administration is repeated it can be carried out by the same route of administration or by different routes of administration. Preferably the route of administration is the same for each delivery.

The pharmaceutical composition according to the invention can be used for the curative (therapeutic) treatment of disorders, in particular of cancer, and preferably of melanoma. However, it can also be used for the preventive treatment (i.e. as a vaccine) in order to prevent the development or progression of disorders, in particular cancer, and preferably of melanoma. Accordingly, the specific design, in particular the dosage, of the different components in the pharmaceutical composition may depend and vary on the specific use.
The choice of the tumor-associated antigen depends, of course, on the type of tumor to be treated. This choice is well within the skill of the person skilled in the art. In particular, the following types of tumors display the following tumor-associated antigens:

| | |
|---|---|
| melanoma | gp100, tyrosinase, MAGE-1, MAGE-3, MART, BAGE, TRP-1 |
| stomach cancer | CEA (carcino embryonic antigen), CA 19-9, CA 50, CA 72-4 |
| Colon cancer | CEA, CA19-9, Muc-1 |
| pancreas carcinoma | CA 19-9, Ca-50, CEA |
| small cell lung cancer | CEA, NSE (neuron specific enolase), EGF-receptor |
| lung cancer | CEA |
| liver carcinoma | α-fetoprotein (AFP) |
| prostata cancer | PSA |
| gall bladder cancer | CA 19-9 |
| Squamous cell carcinoma | SSC (squamous cell carcinoma antigen), CEA |
| Mammary carcinoma | CA 15-3, CEA, BRCA-1, BRCA-2, Muc-1, Her2/Neu receptor |
| Testes cancer | AFP, hCG |
| ovarial carcinoma | CA-125, CEA, CA 15-3, AFP, TAG-72 |
| B cell lymphoma | CD20, CD21 |

Preferably, the cancer to be treated or prevented is melanoma and the tumor-associated antigen is a gp100 protein.

The present invention also relates to the use of a nucleic acid molecule encoding a tumor-associated antigen in combination with at least one peptide comprising a region corresponding to a putative cytotoxic T cell epitope of a tumor-associated antigen, preferably of the same tumor-associated antigen as that encoded by the nucleic acid molecule and/or cells pulsed in vitro with said at least one peptide for the preparation of a pharmaceutical or vaccine composition for the treatment or prevention of cancer.
The cancer to be treated or prevented may be any type of cancer for which a tumor-associated antigen is known. Examples are those types of cancer listed above.

With respect to the preferred embodiments of the nucleic acid molecule, the peptide(s), the cells, the design of the pharmaceutical or vaccine composition, etc., the same applies as has been set forth above in connection with the pharmaceutical or vaccine composition according to the invention.
In a particularly preferred embodiment the tumor-associated antigen is a gp100 protein and the cancer to be treated is melanoma.

Finally, the present invention also relates to a method for preventing or treating cancer in an individual characterized in that a nucleic acid molecule encoding a tumor-associated antigen is administered in combination with at least one peptide comprising a region corresponding to a putative cytotoxic T cell epitope of a tumor-associated antigen, preferably of the same tumor-associated antigen as that encoded by the nucleic acid molecule, and/or with cells pulsed in vitro with such at least one peptide, and wherein the nucleic acid molecule, peptide(s) and/or cells are administered in a pharmaceutically effective amount.

The nucleic acid molecule and the peptide(s) and/or cells may be administered simultaneously, in parallel or sequentially in any possible order.
Typically, a therapy consists of sequential administration of DNA and peptide. A typical treatment comprises application of DNA at day 0 and then two to four immunizations at two-week of three-week intervals. Peptide boosts are preferably first applied two weeks after the last DNA vaccination and are repeated 3 times at one-week intervals. The peptides are preferably administered by ex vivo treatment by pulsing of autologous DC. The dose of DNA may vary, e.g., from 10 to 250 µg. The doses of peptides may vary, e.g., from 50 to 200 µg for pulsing of 1-5 x 10⁶ DCs.

With respect to the preferred embodiments of the nucleic acid molecule, the peptide(s), the cells, the design of the pharmaceutical or vaccine composition, etc., the same applies as has been set forth above in connection with the pharmaceutical composition according to the invention.

In a particularly preferred embodiment the tumor-associated antigen is a gp100 protein and the cancer to be treated or prevented is melanoma.

These and other embodiments are disclosed and obvious to a skilled person and embraced by the description and the examples of the present invention. Additional literature regarding one of the above-mentioned methods, means and applications, which can be used within the meaning of the present invention, can be obtained from the state of the art, for instance from public libraries for instance by the use of electronic means. This purpose can be served inter alia by public databases, such as the "medline", which are accessible via internet, for instance under the address http://www.ncbi.nlm.nih.gov/PubMed/medline.html. Other databases and addresses are known to a skilled person and can be obtained from the internet, for instance under the address http://www.lycos.com. An overview of sources and information regarding patents and patent applications in biotechnology is contained in Berks, TIBTECH 12 (1994), 352-364.
All of the above cited disclosures of patents, publications and database entries are specifically incorporated herein by reference in their entirety to the same extent as if each such individual patent, publication or entry were specifically and individually indicated to be incorporated by reference.

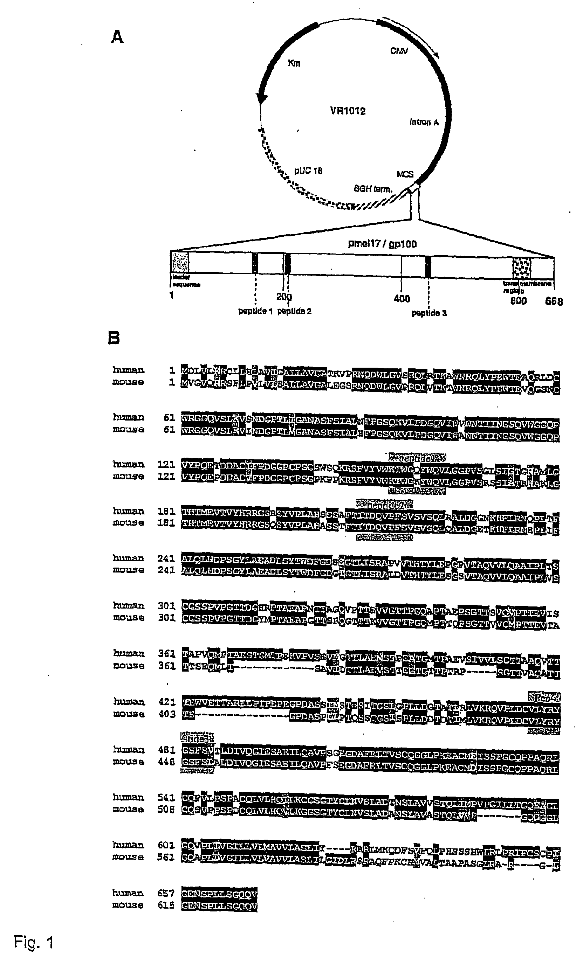

- **Figure 1**: shows the plasmid DNA used for immunization. **A:** The plasmid DNA backbone consists of the CMVi.e. promoter, intron A sequences, bovine growth hormone (BGH) terminator, puc18 sequences and the kanamycin (km) resistance gene. This DNA is usedas empty plasmid control. The human pmel17/gp100 DNA is inserted into a multicloning site (MCS). The gray bars within pmel17/gp100 represent the positions of the three HLA-A2 (MHC class I)-restricted peptides used in this study. **B:** Defined human CTL epitopes within the pmel17/gp100 amino acid sequence. Sequence alignment of human (upper line) and murine gp100 (lower line). Black underlined regions indicate homologous, light gray underlined regions related amino acids. The three peptides from human gp100 used (peptide 1 = KTWGQYWQV, peptide 2 = ITDQVPFSV, peptide 3 = VLYRYGSFSV) are indicated.

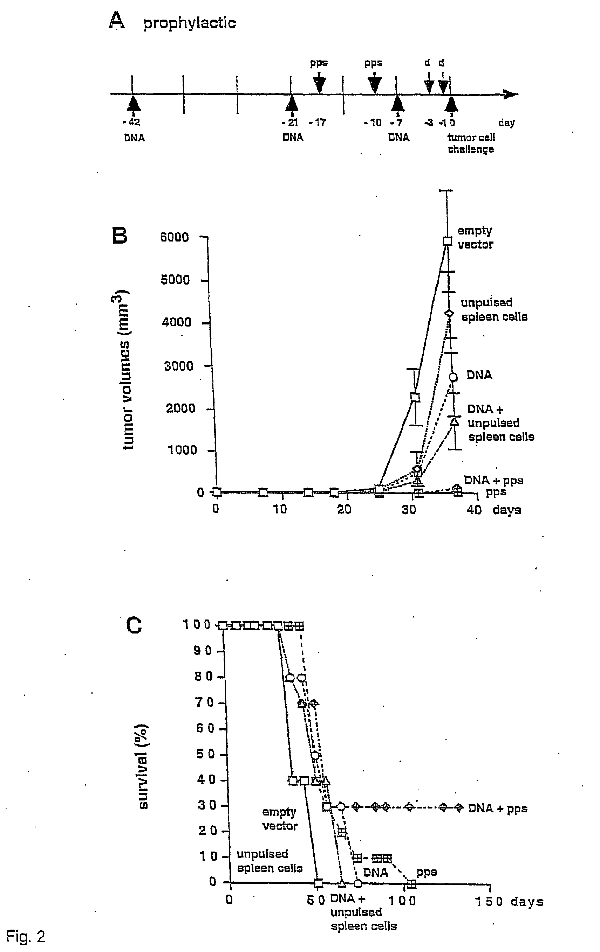

- **Figure 2**: shows the prophylactic vaccination of C57BL/6 mice with DNA and peptides. **A:** The immunization scheme indicates the days of vaccination by arrows. gp100 DNA (100µg per mouse) was injected intramuscularly, peptide-pulsed spleen cells (pps) were applied intraperitoneally. At day 0, 2x10⁴ B16-F0 melanoma cells were injected subcutaneously for tumor cell challenge. Depletions of CD4⁺ and CD8⁺ T cells is indicated by d. **B:** Tumor volumes of mice treated using empty vector ( □ ) and unpulsed spleen cells ( ◇ ) as negative controls, hgp100 DNA ( O ), hgp100 DNA together with unpulsed spleen cells as internal control ( Δ ), pps ( 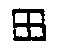 ), and hgp100 DNA together with pps ( 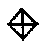 ). Each of the shown curves represents the mean tumor sizes per group (10 mice) of a representative experiment in days after challenge. The standard deviation is indicated as bars within the graph; the significances calculated for pps and the combination of DNA with pps have a probability value of p < 0.01, for unpulsed spleen cells, DNA, and DNA together with unpulsed spleen cells p < 0.05, median test under consideration of a Bonferroni correction. **C:** The corresponding survival curves of the animals are shown.

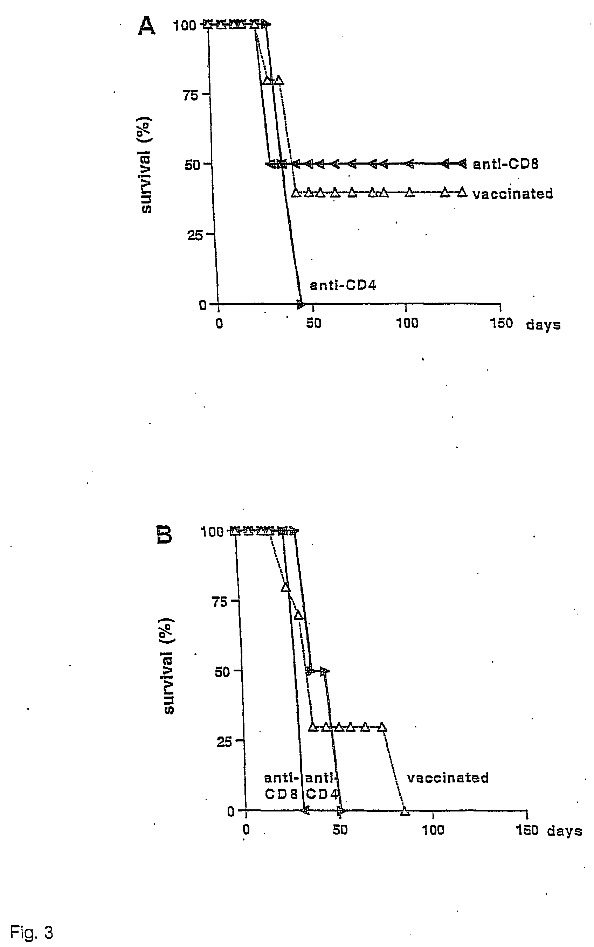

- **Figure 3**: shows survival curves of mice after CD4⁺ and CD8⁺ T cell depletion *in vivo.* **A:** Mice were vaccinated with hgp100 DNA and pps as described in Fig. 2. At day -3 and -1, mice were injected intravenously with purified monoclonal antibodies against CD4⁺ and CD8⁺ T cells or with an irrelevant antibody of the same isotype (1mg per mouse). Two arrows indicate depletion (d) in Fig. 2A. Depletions are indicated by anti-CD4 ( 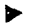 ) and anti-CD8 ( 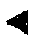 ), the controls are indicated by "vaccinated" (Δ). **B:** Mice for depletion experiments were treated with hgp100 DNA in combination with unpulsed spleen cells in order to evaluate a possible non-specific effect due to the spleen cells. The experiment was exactly performed as in A. Depletion of CD4⁺- ( 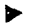 ) and CD8⁺ T cells ( 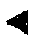 ) and vaccination ( Δ ) is indicated. Survival curves of the animals are shown.

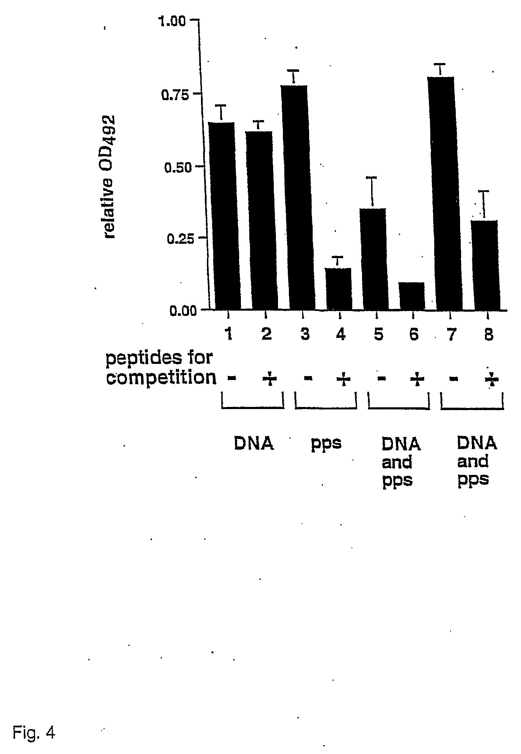

- **Figure 4**: shows the determination of gp100-specific antibody titer in sera of vaccinated mice by ELISA. Mice were bled at the day of tumor cell challenge (see day 0 in Fig. 2A), and long-surviving tumor-free mice vaccinated with DNA together with pps were bled at day 108 (see Fig. 2C). The gp100-specific antibody titers in the mouse sera are given as their relative absorbance at a wave length of 492nm. The gp100 antibody titer of mice either vaccinated with DNA and unpulsed spleen cells is shown in lanes 1 and 2, vaccinated with pps alone in lanes 3 and 4, vaccinated with DNA together with pps in lanes 5 and 6 (mice bled at day 0) and in lanes 7 and 8 (mice bled at day 108). The sera were incubated on ELISA plates coated with cell extracts of B16-F0 melanoma cells in the absence (**-**) and presence of the three gp100 peptides (**+**). The relative absorbance obtained by sera of control mice was subtracted as background level. Each measurement was performed in duplicate. The standard deviations are indicated as error bars, the deviation in lane 6 was too small to generate an error bar.

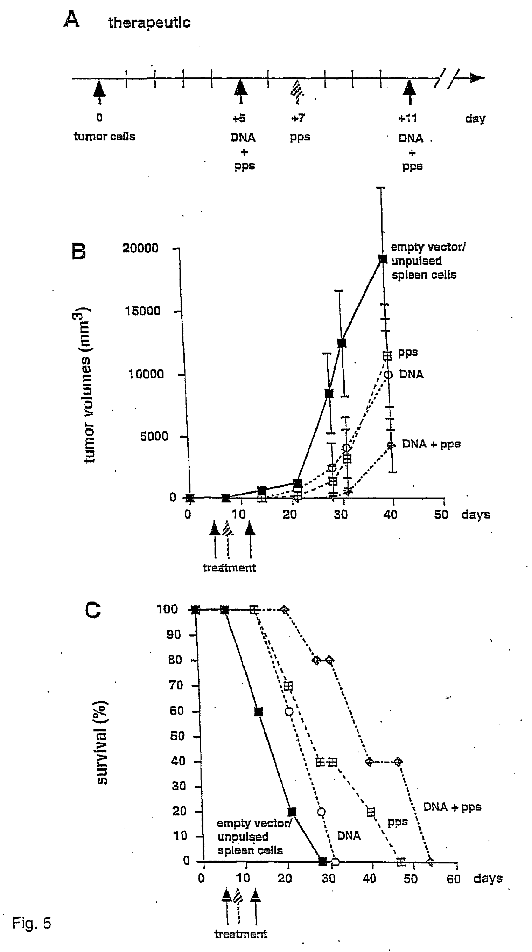

- **Figure 5**: shows the therapeutic vaccination with DNA and peptides *in vivo.* **A** shows the immunization scheme. At day 0, tumor cells (2x10⁵ B16-F0) were inoculated subcutaneously. Afterwards at the days indicated gp100 DNA (100µg), pps (2x10⁷), or both were applied. **B:** Tumor volumes of animals treated with empty vector DNA together with unpulsed spleen cells (■ ), pps alone ( 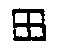 ), hgp100 DNA alone ( ○ ), and combination of hgp100 DNA with pps ( 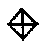 ) were determined. Mean tumor sizes per experimental group (ten C57BL/6 mice) of a representative experiment are depicted in time after challenge generating tumor growth curves. The standard deviation is indicated as bars within the graph; the significances calculated for DNA together with pps are p < 0.01, and for pps alone and DNA alone p < 0.05 - median test under consideration of a Bonferroni correction. The two black arrows on the x-axis indicate the combinatory treatment with DNA and pps, the dotted arrow indicates the pps treatment. **C:** Corresponding survival curves of the animals are shown.

The following Examples further illustrate the invention.

In the Examples the following materials and methods were used.

### Materials and Methods

### 1. Construction of plasmids for immunization and expression control

A cDNA fragment encoding human gp100 (*Sall-Notl* fragment) was inserted into the multiple cloning site of the expression vector VR1012 (Vical Co., San Diego, USA) as described in Nawrath et al. (Adv. Exp. Med. Biol. 451 (1998), 305-310 and Moelling et al. (in. "Strategies for Immunointerventions in Dermatology" (1997), Springer Verlag, 195-206). Plasmid purification and control for expression of the hgp100 protein using the monoclonal antibody HMB45 for immunofluorescence were performed as described in Nawrath et al. ((1998), loc. cit.).

### 2. Cell line and mice

B16-F0 melanoma cells were maintained in Dulbecco's modified Eagle's medium (DMEM) supplemented with 10% FCS, penicillin G (100 units/ml), streptomycin sulfate (100µg/ml) and 2mM L-glutamine. The expression of hgp100 antigen was verified in B16-F0 tumor cells by immunofluorescence analysis.
For all experiments 8 to 10-week old C57BL/6 mice were used. Implantation of subcutaneous tumors was carried out by injection of B16-F0 melanoma cell suspensions (2x10⁴ for the prophylactic approach and 2x10⁵ for the therapeutic approach). The resulting tumor volumes were calculated as previously described (Pavlovic et al., Gene Therapy 3 (1996), 635-643). For statistical analysis, the median test under consideration of a Bonferroni correction was used.

### 3. Preparation of autologous peptide-pulsed spleen cells and vaccination of mice

After the surgical removal of spleens from naive C57BL/6 mice, the spleens were crushed through a sterile grid directly into a dish containing DMEM medium supplemented with 2% FCS. The cells were vigorously suspended, transferred into Falcon tubes, and centrifuged for a few seconds after which the tubes were let stand for 10 minutes. Single spleen cells remained in the supernatant, which was poured off into another tube. The leukocytes were counted by trypan-blue exclusion and adjusted to a cell density of 7x10⁶ viable cells per ml. All three peptides used for pulsing (peptide 1 KTWGOYWQV (SEQ ID NO:5), peptide 2 ITDQVPFSV (SEQ ID NO:6) and peptide 3 VLYRYGSFSV (SEQ ID NO:7)) were purchased from MWG Biotech (Ebersberg, Germany). A 100-fold peptide stock solution in PBS was prepared using several sonification steps to promote dissolution. The peptide solution (10 nM) was added to the spleen cell suspension and incubated for 1 hour at room temperature and for another 1 hour at 37°C under 5%CO₂. The peptide-pulsed spleen cells were injected intraperitoneally into the mice (2x10⁷ cells per mouse).

### 4. CD4⁺ - and CD8⁺ T cell depletion of mice in vivo

The hybridomas as source for the rat anti-CD4 and anti-CD8 antibodies were grown in mass cultures and the IgG fractions purified using protein-G sepharose. The mice for these experiments were depleted of CD4⁺- and CD8⁺ T cells by intravenous injection of 1mg purified anti-CD4 antibody GK1.5 (Dialynas et al., J. Immunol. 131 (1983), 2445-2451) or anti-CD8 antibody 2.43 (Sarmiento et al., J. Immunol. 125 (1980), 2665-2672) respectively into the tail vein three days and one day before tumor cell challenge. Depletion efficiency was checked 1 day and 1 and 2 weeks after depletion by FACS analysis of peripheral blood. CD4⁺- and CD8⁺ T cells were below the detection level every time the mice were bled. As control, the respective vaccinated mice were treated with an irrelevant monoclonal antibody of the same isotype.

### 5. ELISA with sera of immunized mice

Sera of immunized mice were taken at the day of tumor cell challenge (see day 0 in Fig. 2A). In addition, sera from long-term surviving mice vaccinated with DNA plus pps were taken at day 108. An ELISA was performed to detect gp100 antibody level as described elsewhere (Pavlovic et al., (in press)). Briefly, ELISA plates in a 96-well format were coated with B16-F0 melanoma cells as target. Different serum dilutions were incubated on freshly coated ELISA plates. For specific competition, sera were preincubated with a 100ng concentrated mixture of all three peptides used. A secondary anti-mouse horseradish peroxidase-coupled antibody was used for the substrate reaction. The absorbance measured at a wavelength of 492nm is proportional to the hgp100-specific antibody titer within the sera. The background level yielded either by sera of naive mice or control DNA vaccinated mice was substracted from each measured gp100 antibody titer. Each measurement was carried out in duplicate.

### Example 1

### Construction of an expression vector for hgp100 and selection of peptides

The previously described expression plasmid VR1012 was used for intramuscular injection into mice (Moelling et al. (1997), loc. cit.). Expression of the transgene is driven by a combination of the CMV immediate early promoter/enhancer region, sequences of the intron A and the bovine growth hormone (BGH)-terminator signal on a puc18 bacterial plasmid backbone also containing a kanamycin-resistance gene. The hgp100 cDNA was inserted into the multiple cloning site as shown in Fig. 1A. The hgp100 gene encodes a 668 amino acid protein with a leader sequence and a single transmembrane domain (Fig. 1A).

Five different immunogenic peptides from the hgp100 protein have been identified previously and shown to be reactive with different hgp100 TILs (Kawakami et al., J. Immunother. 21 (1998), 237-246). Two of the nonamer peptides which were also used in the present application, peptide 1 G9(154-162) and peptide 2 G9(209-217), appeared to be immunodominant and were widely recognized by TILs (Pass et al., Cancer J. Sci. Am. 4 (1998), 316-323; Rivoltini et al., J. Immunol. 156 (1996), 3882-3891; Salgaller et al., Cancer Res. 56 (1996), 4749-4757; Clay et al., J. Immunol. 162 (1999), 1749-1755). Peptide 3, the decamer G10(476-485), is expressed on the surface of melanoma cells and is able to induce melanoma-reactive CTLs from peripheral blood lymphocytes (PBL) of melanoma patients by repeated *in vitro* stimulation (Salgaller et al., Cancer Res, 55 (1995), 4972-4979). Thus, these gp100 epitopes were considered as good candidates for use in peptide-based immunotherapies. Recently, treatment of melanoma patients with synthetic peptides representing putative CTL-specific epitopes summarized in a review of Rosenberg ((1997), loc. cit.) have shown some success when used for *ex vivo* pulsing of autologous APCs, for instance dendritic cells which are specialized for the induction of primary T cell response (Nestle et al., Nat. Med. 4 (1998), 328-332). Three of previously described CTL epitopes used for pulsing autologous spleen cells from C57BL/6 mice are indicated in the sequence alignment of human and mouse gp100 (Fig. 1B), peptide 1 (KTWGOYWQV; SEQ ID NO:5), peptide 2 (ITDQVPFSV; SEQ ID NO:6) and peptide 3 (VLYRYGSFSV; SEQ ID NO:7). Peptide 2 is identical between human and mouse, while the peptides 1 and 3 differ in one amino acid.

### Example 2

### Prophylactic vaccination

In order to analyze the effect of gp100 DNA as a vaccine in combination with peptides, a vaccination protocol was designed as shown in Fig. 2A. gp100 DNA (100 g) was injected intramuscularly three times, at days -42, -21 and -7. Peptide-pulsed spleen cells were applied twice at day -17 and -10 before tumor cell challenge. The three peptides were mixed and added directly into the medium containing freshly isolated autologous spleen cells from naive C57BL/6 mice. The pulsing of spleen cells was performed twice, each time for one hour, and 2x10⁷ of pulsed cells per mouse were injected intraperitoneally. At day 0, B16-F0 melanoma cells (2x10⁴) were injected subcutaneously into syngeneic C57BL/6 mice. Subcutaneous injection of 2x10⁴ cells gives rise to tumors of about 2000mm³ within 30 days. Always a total of 10 mice was used for each experimental group. Mice were treated with either hgp100 DNA or peptide-pulsed spleen cells (pps) or a combination of the two. Controls comprised DNA without hgp100 insert, unpulsed spleen cells, and hgp100 DNA together with unpulsed spleen cells to evaluate an unspecific effect of the spleen cells on the hgp100 DNA vaccination effect. The control experiment using plasmid DNA without hgp100 insert together with pps was not included because an immunostimulatory effect of the VR1012 empty vector was never observed in previous studies (Nawrath (1998), loc. cit.; Nawrath (1999), loc. cit.).
As shown in Figure 2B, vaccination with DNA encoding gp100 without or with unpulsed spleen cells resulted in a two to threefold reduction of the tumor growth (2000 to 2800 mm³) compared to controls (5800 mm³, empty vector). Animals treated with pps developed only very small tumors (<100 mm³) within the same time frame (37 days post tumor cell challenge). Subsequently, however, these mice rapidly developed tumors (data not shown) and died within 70 days post challenge (Fig 2C). Vaccination of mice with a combination of DNA encoding hgp100 and pps yielded a synergistic effect. 30% of the animals remained tumor free over the entire observation period of 133 days (Fig 2B and C). The other animals of this vaccination group showed similar tumor growth kinetics as mice treated with pps alone. They developed only very small tumors (<100 mm³) by 37 days post tumor challenge but like the pps-treated group showed rapid tumor growth resulting in the death of 90% of the mice within 70 days.

### Example 3

### CD4⁺ and CD8⁺ T cell depletion in vivo

To assess the contributions of CD4⁺- and CD8⁺ T cells to the protective effect of the vaccination, these cells were depleted at day -3 and day -1 before tumor cell challenge (see Fig 2A, "d"). Depletion was performed as previously described by using monoclonal antibodies against CD4 and CD8, respectively (Dialynas et al., loc. cit.; Sarmiento et al., loc. cit.). The survival rates of depleted mice were compared with those of the vaccinated ones (Fig. 3A and B). CD4-depleted mice, vaccinated with DNA and pps, did not benefit from vaccination. The survival rate of all of these mice rapidly declined until day 44 (Fig. 3A). CD8-depletion gave similar results with 50% of surviving animals as observed for the undepleted ones with 40% of surviving animals (Fig. 3A). The results of this vaccination regime suggest, that CD4⁺ T cells are absolutely essential for antitumor activity.
An analogous experiment was performed with mice vaccinated by gp100 DNA in combination with unpulsed spleen cells. Under these conditions, CD8⁺ cells were absolutely essential as might be expected from a DNA antitumor vaccine. CD4⁺ T cells were also required to a similar extent (Fig. 3B). The survival rates of the CD8-and CD4-depleted mice dropped at day 37 and 51, respectively. The mice vaccinated with DNA and unpulsed spleen cells survived until day 85 only. Comparison of Fig. 3A and B indicates the effect that can be attributed to the peptides. Hence, vaccination with pps shifts the CD8-dependent protection induced by vaccination with hgp100 DNA alone to a CD4-dependent protection for the DNA-pps combination.

### Example 4

### CD4-dependence and humoral immune response in vaccinated mice

Mice vaccinated with DNA and unpulsed spleen cells developed a gp100 antibody titer that could not be competed with the three gp100 peptides (see lanes 1 and 2 in Fig. 4). This result suggests that the entire gp100 DNA sequence comprises additional, as yet unidentified gp100-specific epitopes. Therefore, a significant specific competition with only the three peptides used in this study cannot be achieved. However, Figure 4 also shows that the high gp100-specific antibody titer in sera taken from the group of mice vaccinated either with pps alone (see lanes 3 and 4) or with DNA in combination with pps (see lanes 5 and 6) could be significantly competed with these three gp100 peptides. The long-term surviving mice vaccinated with DNA together with pps reveal a persistent and even higher gp100 antibody titer compared to the titer observed at the day of tumor cell challenge (compare lane 5 to lane 7). This result underlines that pps are required to achieve persistent high-titer antibodies in this antitumor vaccine approach.

### Example 5

### Therapeutic vaccine

More relevant for treatment of tumor diseases by vaccines is a therapeutic potential of vaccines. Especially in the case of malignant melanoma therapeutic possibilities are rather limited. Therefore, a therapeutic vaccination protocol was designed as shown in Fig. 5A. At day zero, ten times more (2x10⁵) B16-F0 melanoma cells than used in the vaccination approach were injected subcutaneously into syngenetic C57BL/6 mice. All the mice would die of tumors of approximately 20.000 mm³ within 40 days. A single treatment of mice consists of injection of hgp100 DNA combined with pps twice at days 5 and 11, and pps alone once at day 7 as indicated (Fig. 5A). This time schedule is based on data (Kundig et al., Proc. Natl. Acad. Sci. USA 93 (1996), 9716-9723; Kundig et al., Immunol. Rev. 150 (1996), 63-90) and personal communication (Dr. TM Kündig, Department of Dermatology, University Hospital of Zurich) concerning the development and maintenance of a specific T cell response. The tumor volumes of mice treated with both DNA and pps was strongly reduced whereas mice treated only with DNA (days 5 and 11) or pps (days 5, 7 and 11) showed significantly higher tumor volumes (Fig. 5B). Similarly, 100% of the mice treated with DNA and pps survived after 28 days, at which time all of the control mice had died. The survival rate of the mice treated with hgp100 DNA alone or with pps alone rapidly declined in parallel to the control (Fig. 5C). Vaccination with only a single round of treatment, based on the combination of DNA and pps, increased the survival of animals about twofold.

### Conclusion from the Experimental results

In the above described Examples it was examined whether a prime/boost approach could be exploited to produce a protective antitumor immunity. Using the mouse B16 melanoma model, it was shown that combining a DNA vaccine against the melanoma-associated antigen pmel17/gp100 with a peptide vaccine derived from the same antigen is effective in both, a prophylactic as well as a therapeutic, setting and superior to either vaccination alone.
For the generation of a DNA vaccine against mouse gp100 the cDNA encoding the human gp100 instead of the mouse gp100 was used. The decision for a xenoimmunization approach was based on previous reports demonstrating mgp100 is poorly immunogenic and vaccination with viral and non-viral vectors expressing mgp100 did not elicit an immune response or protective immunity in mice against B16 melanoma (Overwijk et al., J. Exp. Med. 188 (1998), 277-278; Zhai et al., loc., cit.). However, xenoimmunization of mice with vectors expressing the hgp100 cDNA yielded specific T cell responses against the human gp100 as well as its mouse homologue mgp100 and, as a consequence, led to a partial protective effect against challenge with B16 cells (Overwijk et al., loc. cit.; Zhai et al., loc. cit.). The human and mouse gp100 are highly homologous proteins of 662 and 626 amino acids, of which 498 are identical. For the peptide vaccination the three human gp100 derived peptides G9(154-162), G9(209-217), and G10(476-485) were selected. These peptides were previously shown to induce HLA-A2 restricted, T cell-specific responses in humans (Salgaller et al., loc. cit.). The sequences of these three epitopes are highly conserved between the human and the mouse gp100 (see Fig 1B). The use of multiple peptides has the advantage of overcoming the HLA-independent heterogeneity of immune responses to individual peptides and antigens in patients with the same haplotype and maximizes the proportion of responding patients (Reynolds et al., J. Immunol. 161 (1998), 6970-6976).
Presentation of tumor antigens by APCs is crucial for the induction of an efficient antitumor immunity. Indeed, direct targeting of peptides to spleen derived APCs or dendritic cells (DCs) by *ex vivo* pulsing has been shown to elicit a more potent immune response than intradermal or intramuscular injection of peptides (Kundig et al., Proc. Natl. Acad. Sci. USA 93 (1996), 9716-9723; Kundig et al., Immunol. Rev. 150 (1996), 63-90; Ikeda et al., Proc. Natl. Acad. Sci. USA 94 (1997), 6375-6379; Fallarino et al., Int. J. Cancer 80 (1999), 324-333). Since APCs from spleen cells are easier to obtain in sufficient numbers than DCs, spleen cells were used for *ex vivo* pulsing with peptides.
Mice vaccinated with a plasmid expressing the hgp100 showed reduced tumor growth upon subcutaneous challenge with B16-F0 cells expressing endogenous mouse gp100. This effect is most likely due to the generation of a cellular immune response, since depletion of CD8⁺ or CD4⁺ T cells using CD8- and CD4-specific antibodies completely abrogated the anti-tumor response (Fig. 3B). Moreover, mice vaccinated with plasmid DNA encoding hgp100 produced antibodies against hgp100 that cross-reacted with mgp100 (Fig. 5). These results clearly demonstrate that an autoreactive immune response against mgp100 can be elicited when the hgp100 is employed as an antigen, confirming reports by Zhai, Overwijk and coworkers (Overwijk et al., loc. cit.; Zhai et al., loc. cit.). However, other studies have failed to demonstrate the generation of cross-reactive T cells to mouse gp100 or protective immunity against challenge with B16 melanoma cells after genetic vaccination with human gp100 (Scheurs et al., loc. cit.; Yang et al., Int. J. Cancer 83 (1999), 532-540). One possible explanation for the observed discrepancies regarding protection among different studies might be the level of expression of endogenous mouse gp100 in B16 cells. In the present study a strong variation of gp100 expression in B16-F0 cells that were obtained from different sources was observed. Therefore endogenous gp100 expression in B16-F0 cells used for tumor challenge experiments was frequently monitored (data not shown).
In both, the prophylactic and the therapeutic approaches, vaccination with a combination of DNA and peptides was clearly more effective than either approach alone (Figs. 2 and 5). In contrast to vaccination with DNA alone, immunization with a combination of DNA and peptide led to a protective immune response that was solely dependent on the presence of CD4⁺ T cells. Depletion of the CD8⁺ T cells had no effect at all in this experimental setting. This suggests that vaccination with the peptides in addition to DNA encoding the entire protein let to a shift from a CD4⁺ and CD8⁺ T cell-dependent to a strictly CD4⁺ T cell-dependent protective immune response. In this context it is interesting to note that the protective effect of the combined treatment with plasmid DNA encoding hgp100 together with pps can be abolished by co-injection of DNA encoding mouse IL-12 (J. Schultz and K. Moelling, unpublished data), a known inducer of a T helper type 1 (Th1) response, indicating that the effect is Th1-independent. Vaccination with the peptides induced the production of peptide-specific antibodies that efficiently cross-reacted with the mouse gp100 (Fig. 4). Interestingly, the cross-reactive gp100 specific antibodies raised in response to the DNA vaccination alone, recognized predominantly epitopes that were not competed for by the peptides, while vaccination with a combination of DNA and peptides produced antibodies that were predominantly directed against the epitopes represented by the peptides. Apparently, vaccination with DNA encoding human gp100 induced a variety of antibodies that recognized several epitopes common to human and mouse gp100. Boosting of this primary response with peptide-pulsed spleen cells then led to a selective production of antibodies directed against the peptides. Vaccination with peptide-pulsed spleen cells alone also elicited a strong antibody response against mouse gp100, despite the fact that the peptide sequences and the corresponding sequences in mouse gp100 largely overlap. However, the small amino acid sequence differences in two out of three epitopes might be sufficient to elicit the observed immune response. Whether the antibodies that cross-react with mouse gp100 play a role in the observed protective effect against the B16 tumor cells remains to be determined. As discussed by Overwijk et al. (loc. cit.), it is conceivable that B cells expressing the antibodies on their cell surface could capture human gp100 or mouse gp100 released from apoptotic or necrotic cells and present them after processing on MHC class II molecules to CD4⁺ T cells (Simitsek et al., J. Exp. Med. 181 (1995), 1957-1963). Alternatively, specific antibodies could recruit gp100 as immunecomplexes to dendritic cells via Fc receptors and present it by MHC class II molecules to naive CD4⁺ T cells. However, Schreurs and coworkers have demonstrated in a similar study that gp100 specific antibodies raised in response to DNA vaccination against gp100 exerted no protective effect upon adoptive transfer to nonvaccinated mice (Schreurs et al., loc. cit.).
It is therefore highly likely that the protective effect is induced by presentation of the peptides on MHC class II molecules of APCs to CD4⁺ T cells which in response become activated and mediate peptide-specific killing of B16 melanoma cells that present endogenous gp100 epitopes on MHC class II molecules. The B16-F0 cells used for these experiments express MHC class II molecules on their cell surface in the presence of IFN type I or II (I. Peter and S. Hemmi, personal communication). Killing of the B16-F0 cells could be due either to the direct cytolytic activity of CD4⁺ T cells or to a cytokine-mediated event with or without the involvement of natural killer (NK) cells and / or macrophages.

Taken together, it was demonstrated that vaccination with DNA encoding the human melanoma-associated antigen gp100 in combination with three gp100-derived peptides yield a protective effect against B16 melanoma that is superior to treatment with either DNA or peptides alone. Interestingly, the protective immunity induced by treatment with pps alone or DNA in combination with pps appears to be primarily mediated by CD4⁺ T cells. These findings suggest that in addition to CD8⁺ T cell-mediated killing of tumor cells, CD4⁺ T cell-mediated immunotherapies with MHC class II specific peptides should be considered for effective treatment.

## Claims

1. A pharmaceutical composition comprising a nucleic acid molecule encoding a tumor-associated antigen and at least one peptide comprising a region corresponding to a putative cytotoxic T cell, helper T cell or B cell epitope of a tumor-associated antigen and/or cells pulsed with such peptide(s).

2. The pharmaceutical composition of claim 1 wherein the tumor-associated antigen encoded by the nucleic acid molecule is heterologous with respect to the species to which the individual belongs to whom the pharmaceutical composition shall be administered.

3. The pharmaceutical composition of claim 1 or 2, which is for administration to humans and in which the nucleic acid molecule encodes a non-human tumor-associated antigen.

4. The pharmaceutical composition of any one of claims 1 to 3, in which the nucleic acid molecule encoding the tumor-associated antigen is under the control of the CMV early promoter.

5. The pharmaceutical composition of any one of claims 1 to 4, in which the nucleic acid molecule is a double stranded circular or linear molecule.

6. The pharmaceutical composition of any one of claims 1 to 5, in which the nucleic acid molecule is naked DNA.

7. The pharmaceutical composition of any one of claims 1 to 6, wherein the tumor-associated antigen is a gp100 protein.

8. The pharmaceutical composition of claim 7, in which the peptide(s) comprise(s) at least one of the following amino acid sequences:
(i) KTWGQYWQV (SEQ ID NO:5);
(ii) ITDQVPFSV (SEQ ID NO:6);
(iii) VLYRYGSFSV (SEQ ID NO:7); and
(iv) KTWGKYWQV (SEQ ID NO:8).

9. The pharmaceutical composition of any one of claims 1 to 8, which comprises more than one peptide comprising a region corresponding to a putative cytotoxic T cell, helper T cell or B cell epitope of a tumor-associated antigen, said peptides having the same or different amino acid sequences.

10. The pharmaceutical composition of any one of claims 1 to 9, which is for the administration to humans and in which the peptide(s) is (are) derived from a non-human tumor-associated antigen.

11. The pharmaceutical composition of any one of claims 1 to 10, in which the peptide-pulsed cells are dendritic cells.

12. The pharmaceutical composition of claim 11, wherein the dendritic cells are derived from the same individual to whom the pharmaceutical composition shall be administered.

13. Use of a nucleic acid molecule encoding a tumor-associated antigen in combination with at least one peptide comprising a region corresponding to a putative cytotoxic T cell, helper T cell or B cell epitope of a tumor-associated antigen and/or cells pulsed in vitro with said at least one peptide for the preparation of a pharmaceutical composition for the treatment or prevention of cancer.

14. The use of claim 13, wherein the tumor-associated antigen is a gp100 protein and the cancer is melanoma.
